Europäisches Patentamt

European Patent Office

Office européen des brevets

(11) Publication number: **0 452 894 A2**

# EUROPEAN PATENT APPLICATION

(21) Application number: 91106111.7

(22) Date of filing: 17.04.91

(51) Int. Cl.5: **C12N 15/86**, C12N 15/36, C12N 15/54

(30) Priority: 19.04.90 IT 2007290

(43) Date of publication of application:
23.10.91 Bulletin 91/43

(84) Designated Contracting States:
AT BE CH DE DK ES FR GB GR LI LU NL SE

(71) Applicant: **CONSIGLIO NAZIONALE DELLE RICERCHE**
**Piazzale Aldo Moro, 7**
**I-00185 Roma(IT)**

Applicant: **ISTITUTO SUPERIORE DI SANITA**
**299 Viale Regina Elena**
**I-00161 Roma(IT)**

(72) Inventor: **Negrini, Massimo**
**Via Borgopunto, 279**
**I-44100 Ferrara(IT)**
Inventor: **Rimessi, Paola**
**Via Comacchio, 969**
**I-44100 Ferrara(IT)**
Inventor: **Manservigi, Roberto**
**Via Ravenna, 595**
**I-44040 Gaibana (Ferrara)(IT)**
Inventor: **Barbanti-Brodano, Giuseppe**
**Via Farini, 26**
**I-40124 Bologna(IT)**

(74) Representative: **Moretti, Giorgio et al**
**Notarbartolo & Gervasi s.r.l. Viale Bianca Maria, 33**
**I-20122 Milano(IT)**

(54) **Human cells for the high expression of genes inserted in episomal recombinant DNA, their preparation and their use.**

(57) Modified human cells are described which allow the expression of products encoded by any gene under the control of HIV-1 LTR.

EP 0 452 894 A2

Rank Xerox (UK) Business Services

FIG. 2

2

Field of the invention

The present invention refers to human 293 cells the cellular genome of which was modified by integration of pSU3Rtat and pHEBo vectors and which were later transformed with episomal vectors pRPneoU3RX, where X̄ represents a gene under the control of HIV-1 LTR, and to a process for their preparation.

State of the art

Normally the expression of exogenous genes in eukaryotic cells is performed utilising rodent or monkey cells, less frequently human cells, which are transformed with integration vectors consisting of retroviruses [Cepko, C.L.; Roberts, B.E.; Mulligan R.C.: Construcions and applications of a highly transmissible murine shuttle vector. Cell 37: 1053-1062 (1984)] or based on virus SV40 sequences [Elder, J.t.; Spritz, R.A.; Weissman, S.M.: Simian virus 40 as a eukaryotic cloning vehicle. Ann.Rev. Genet. 15: 295-340 (1981)] and on the bovine papilloma sequences. [DiMaio, D.: Papillomavirus cloning vectors. The papillomaviruses (ed Howley, P.M. and Salsman, N.P.), Plenum Press, New York, p.293 (1987)]. This last may remain in the episomal state and amplify itself in mouse cells. The production obtained with the aid of said cells is variable and not always very high. Furthermore, particularly in retrovirus transformed cells, exogenous DNA frequently rearranges with ensuing loss of expression. According to the present invention it is possible to utilize instead human cells, and a episomal vector is employed which is capable of amplification and to alternatively present constitutive or inducible expression through the HIV LTR transactivation by tat. The constitutive expression is obtainable in 293 tat cells, the enducible expression in normal 293 cells which are transitorily transfected with tat expressing plasmides.

Detailed description of the invention

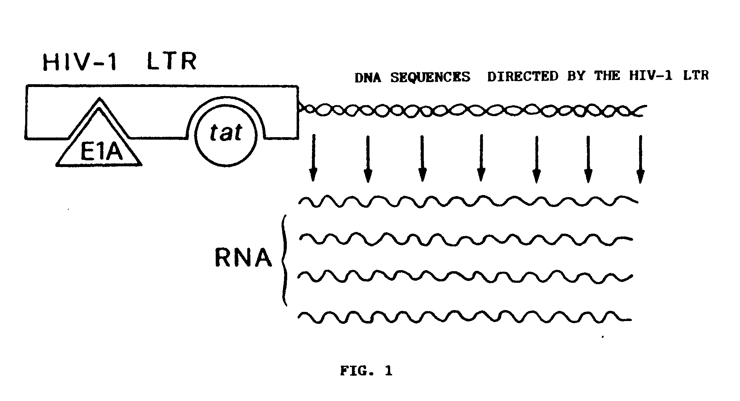

The HIV-1 LTR (the virus which is the etiological agent of AIDS) containing the transcription promotor-enhancer, is activated by a series of transcriptional factors which recognize different sequences and the effect of which is additive. Two of the strongest transactivating factors are E1A, a precocious adenovirus protein, and tat, encoded by a HIV-1 gene (figure 1 where the arrows indicate a strong increase of the RNA transcription due to the effect of E1A and tat on the HIV-1 LTR).

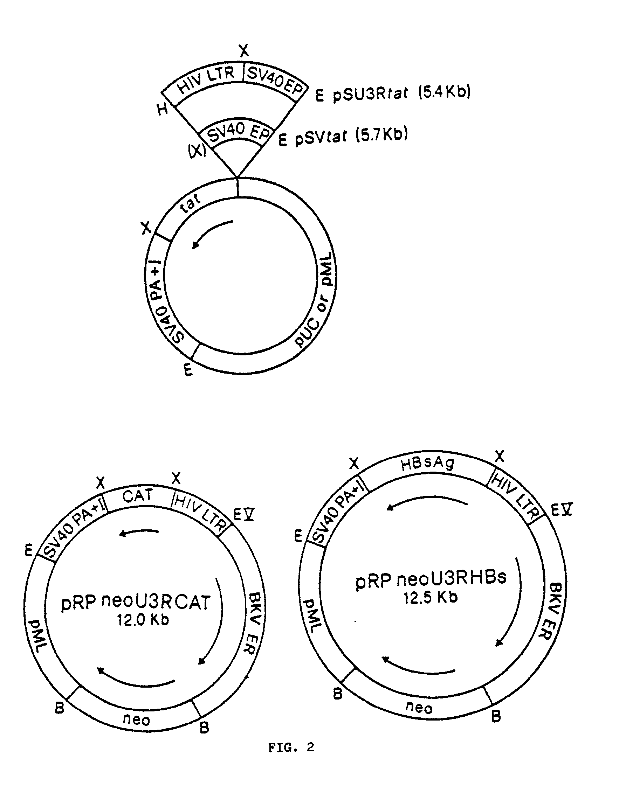

According to the present invention human 293 (ATCC CRL 1573) cells are employed transformed by the precocious region of 5 adenovirus which constitutively express E1A. Said cells were co-transfected with the pSU3R tat integration vector constructed by us (figure 2) and with pHEBo which expresses the resistance to hygromycine B. As a result of this co-transfection, both vectors permanently integrate into the cell genoma so that the transformed cells can be selected in a medium containing hygromycin B and are at the same time able to express tat in a constitutive manner.

Said cells, which now constitutively express EIA and tat were then further transformed with the episomal vectors pRPneoUR3rX built by us, where X represents a gene under the control of the HIV-1 LTR, consisting of recombinant DNA constructed with the BK virus genoma and therefore able to replicate in human cells. In particular, for this operation two vectors were employed in which the bacterial gene for the cloramphenicol acetyl-transferase (CAT) enzyme, that is pRPneoU3HCAT, and the structural gene for the surface antigen of hepatitis B virus (HBsAg), that is pRPneoU3RHBsAg (Figure 2) were inserted. This last is of considerable medical interest because of its possible diagnostical and vaccinal applications.

The selection of the clones transformed after the second transfection was performed with G418, a aminoglycosidic antibiotic to which the cells expressing the neo gene, contained in fact in said episomal vectors, acquire resistance. The products of both genes were expressed at very high levels in 293 tat cells with a conversion, in two minutes, into the different cellular clones, of 98-99% of cloramphenicol into its monoacetylated forms and with HbsAg concentration in the culture medium which reaches 750 ng/ml or 50 ng/$10^6$ cells/24h; which represents aproximately 50 times the production obtained in normal 293 cells. (TABLE 1)

TABLE 1

Production of HBsAg in 293tat cells and normal 293 cells transfected with pRPneoU3RHBs

Short term tests (48 h)

| Cells | HBsAg (ng/ml) |
|---|---|
| 293tat cl1 | 3.8 |
| 293tat cl4 | 5.5 |
| 293tat cl5 | 4.5 |
| 293tat cl6 | 2.7 |
| 293tat cl8 | 5.1 |
| 293tat cl9 | 33.3 |
| 293 normal | 1.2 |

Stable clones

| Cells | HBsAg ng/ml (7d)* | ng/$10^6$ cells/24h | * Production ratio 293tat / 293* |
|---|---|---|---|
| 293tat cl4/1 | 744.0 | 310.6 | 48 |
| 293tat cl4/6 | 90.0 | 27.8 | |
| 293tat cl4/7 | 477.0 | 250.5 | |
| 293tat cl9/2 | 237.0 | 102.9 | |
| 293 cl1 | 4.5 | 0.1 | |
| 293 cl2 | 20.0 | 3.3 | |
| 293 cl3 | 5.0 | 0.2 | |

* In stable clones the HBsAg concentration values were determined in the culture medium after 7 days the seeding of cells

* * The HBsAg production ratio was obtained comparing the average of cells 293tat and 293 clones values

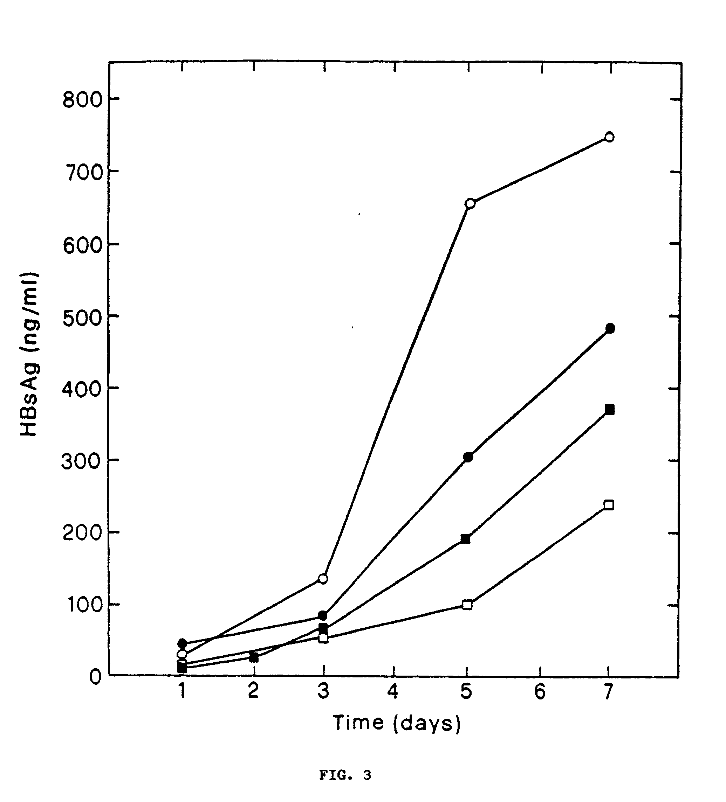

These values are among the highest described up to now in eukaryotic cells systems. Furthermore, the production kinetics shows that after 7 days the HBSag secretion in the medium is still in linear growth (Figure 3 where: O , 293tat 4/pRPneoU3RHBs cl 1; ● , 293tat 4/pRpneoU3RHBs cl 7; □ , 293tat 9/pRPneoU3RHBs cl 2; ■ , 293/pRPRSVHBs cl 507;△,normal 293).

Therefore it is possible to further increase the cumulative antigen production continuing the cell culture beyond the first week. The described system is therefore suitable to many application because of the high expression of products encoded by any gene placed under the control of the HIV-1 LTR. A further advantage is represented by the fact that the expression system is specific for human cells and therefore ensures the maximum genuinity and conformational correspondence of the recombinant products with the natural products to be used in man. Furthermore, seen the use of DNA recombinants constructed with the BK virus genoma, which replicate in human cells, the genic expression is amplified.

The described system can therefore be utilised for the production of reagents for therapeutic, diagnostic and vaccinal purposes.

Experimental part

Vector construction

pRPneoU3R derives from vector pRPneo-c, constructed starting from the pRP-C vector [Grossi, M.P.; Caputo, A; Rimessi, P.; Chiccoli, L.; Balboni, P.G.; Barbanti-Brodano, G.: New BK virus episomal vector for complementary DNA expression in human cells. Arch. Virol. 102:275-283) (1988)] by insertion in the BamHI site of the bacterial neo gene, which imparts to eukaryotic cells resistance to the G418 antibiotic. The pRPneo-c vector was modified by substituting the precocious SV40 promotor, situated among the sites recognized by restriction enzymes XhoI and EcoRV, with the HIV-1 LTR derived from the pU3RIII vector. [Sodroski, J.; Rosen , C.; Wong-Staal, F.; Salahuddin, S.Z.; Popovic, M.; Arya, S; Gallo, R.C.; Haseltine, W.: Transacting transcriptional regulation of human T-cell leukemia virus type III long terminal repeat. Science 227:171-173 (1985)]. Both pRPneoU3RCAT and pRPneoU3RHBs derive from the pRPneoU3R vector by insertion in the unique site XhoI of the bacterial gene for the cloramphenicol acetyl transferase enzyme (CAT) [Alton, N.; Vapnek, D.: Nucleotide sequence analysis of the chloramphenicol resistance transposon Tn9. Nature 282:864-869 (1979)] or of the surface antigen of hepatitis B (HBs) virus subtype adw [Moriarty, A.M.; Hoyer, B.H.; Shish, J.W.; Gerin, J.L.; Hamer, D.H.: Expression of the hepatitis B virus surface antigen gene in cell culture by using am SV40 vector. Proc. Natl. Acad. Sci. U.S.A. 78:2606-2610 (1981)]. As to pSU3R tat, this vector was constructed through several intermediate steps. Initially the HIV-1 LTR obtained from vector pU3RIII [Science 227:171-173 (1985)] and the cDNA encoding for the HIV-1 tat obtained from plasmide pGEMIII]tat, were inserted together in the pRPneo-c vector XhoI site [Arch.Virol. 102:275-283 (1988)] generating the pRPneoSU3Rtat vector. The region contained in the EcoRI which directs the tat expression was then inserted in plasmid pUC19 [Yanish-Peron, C; Vieire, J.; Messing, J.: Improved M13 phage cloning vectors and host strains: nucleotide sequences of the M13mp18 and pUC19 vectors, Gene 33:103-119 (1985)].

To obtain the constitutive product expression, human 293 cells [Graham, F.L.; Smiley, W.C.; Nairn, R.: Characteristics of a human cell line transformed by DNA from human adenovirus type 5. Journal of General Virology 36:59-72 (1977)] were cotransfected utilising the DNA coprecipitation in caltium phosphate [Graham, F.L.; Van der Eb, A.J.: A new technique for the assay of the infectivity of human adenovirus 5 DNA. Virology 52:456-467 (1973) and Wigler, M.; Pellicer, A.; Silverstein, S.; Axel, R.; Urlaub, G.; Chasin, L.: DNA-mediated transfer of the adenine phosphoribosyltransferase locus into mammalian cells. Proc. Nat. Acad. Sci. (USA) 76:1373-1376 (1979)] with integration vectors pSU3Rtat and pHEBo.

This last imparts resistance to hygromycine B: therefore resistant clones were extracted, after selection in a medium containing hygromycin B (200 μg/ml), and propagated in the colture.

The clones expressing tat were individualized by transfection with pTZIIICAT plasmid, in which CAT is directed by the HIV-1 LTR, and enzymatic test for the chloramphenicol acetyltransferase (CAT) activity [Gorman, C.M.; Moffat, L.F.; Howard, B.H.: Recombinant genomes which express chloramphenicol acetyl-transferase in mammalian cells. Mol. Cell. Biol. 2:1044-1051 (1982)].

The tat producing clones were transfected with the pRPneoU3RCAT or pRPneoU3RHBs vector, where genes CAT and HBs are directed by the HIV-1 LTR.

A second clone series was then obtained by expression of the amminoglycoside phosphotransferase, encoded by the neo gene which is present in both plasmids. This enzyme inactivates G418, an antibiotic similar to neomycin which is toxic for eukaryotic cells [Southern, P.J.; Berg, P.: Transformation of mammalian cells to antibiotic resistance with a bacterial gene under control of the SV40 early region promoter. J.Mol.Appl. Genet. 1:327-341 (1982)]. The clones obtained in a medium with added G418 (200 μg/ml were expanded in cell coltures which were then tested by the enzymatic reaction for the CAT expression and by a ELISA test [Corallini,A.; Pagnani,M.; Viadana,P.; Silini, E.; Mottes,M.; Milanesi, G.; Gerna, G.; Vettor, R.; Trapella,G.; Silvani, V.; Gaist, G.; Barbanti-Brodano, G.: Association of BK virus with human brain tumors and tumors of pancreatic islets. Int. J. Cancer 39:60-67 (1987)] for the HBs antigen expression.

The inducible expression was obtained by transfecting normal 293 cells with pRPneoU3RCAT or with pRPneoU3RHBs and selecting the clones in medium with G418. The CAT and HBs antigen expression, constitutevely rather low, was transitorially induced by transfecting the cells with tat expressing vectors (pSU3Rtat, pRPneoU3Rtat or pRPneoSVtat).

**Claims**

1. Human 293 cells the cellular genome of which was modified with pSU3tat and pHEBo integration vectors and which were successively transformed with episomal vectors pRPneoU3RX, where X represents a gene under the control of the HIV-1 LTR.

2. Cells according to claim 1, in which the episomal vector is pRPneoU3RCAT.

3. Cells according to claim 1 in which the episomal vector pRPneoU3RHBs.

4. Episomal vector pRPneoU3RX where CX represents a gene under the control of the HIV-1 LTR.

5. Vector according to claim 4, in which X is CAT.

6. Vector according to claim 4, in which X is HBsAG.

7. Integration vector pSU3Rtat.

8. A process for the preparation of cells according to claims 1-3 in which human 293 cells, transformed by the precocious adenovirus 5 region, are co-transfected with the integration vector pSV3tat and with pHEBo and then transformed with the episomal vector pRPneoU3RX where X is a gene under the control of HIV-1 LTR.

9. Process according to claim 8 in which the episomal vector is pRPneoU3RCAT.

10. Process according to claim 8 in which the episomal vector is RPneoU3RHBs.

11. The acetyl-transferase (CAT) chloramphenicol enzyme produced with the cells according to claim 1.

12. Hepatitis B virus surface antigen (HBsAg) produced with the cells according to claim 1.

## HIV-1 LTR

FIG. 1

EP 0 452 894 A2

FIG. 2

FIG. 3